# EUROPEAN PATENT SPECIFICATION

(11) **EP 4 450 112 B1**
(45) Date of publication and mention of the grant of the patent: **21.10.2026**
(21) Application number: 22907499.2
(22) Date of filing: 15.12.2022
(51) Int. Cl.: A61M 25/098, A61M 25/10

(54) **BALLOON CATHETER**
BALLONKATHETER
CATHÉTER À BALLONNET

(30) Priority: 15.12.2021 JP 2021203088
(43) Date of publication of application: 23.10.2024
(73) Proprietor: Toray Industries, Inc., Tokyo 103-8666 (JP)
(72) Inventor: SATO, Ryota, Otsu-shi, Shiga 520-2141 (JP)
(74) Representative: Kador & Partner Part mbB
(86) International application number: PCT/JP2022/046136
(87) International publication number: WO 2023/112976

(56) References cited:
- WO-A1-2019/156195
- JP-A- 2008 264 569
- JP-A- 2008 264 569
- US-A- 4 955 895
- US-A1- 2005 154 414
- US-A1- 2020 061 352
- US-A1- 2021 045 806

## Description

### TECHNICAL FIELD

The present invention relates to a balloon catheter including a radiopaque marker.

### BACKGROUND ART

In the medical field, balloon catheters are used for minimally invasive treatment, and applications thereof are very diverse, including vascular stenosis, cardiac valve stenosis, arrhythmia, and embolic material removal. A general balloon catheter structure has an outer shaft and an inner shaft that constitute a shaft. A balloon is formed by connecting a proximal end portion of the balloon to a distal end portion (tip portion) of the outer shaft and a distal end portion (tip portion) of the balloon to a distal end portion (tip portion) of the inner shaft. The balloon in the balloon catheter can be expanded or contracted by causing fluid to flow through a channel between the outer shaft and the inner shaft.

In addition, the shaft may include a marker portion that is made of a radiopaque material and enables accurate and prompt positioning to a treatment site when a procedure for expanding an obstructed area is performed using the balloon. For example, the following balloon catheter has been reported in Patent Document 1. In the balloon catheter, the proximal end side of the balloon is connected to the tip portion of the outer shaft, the distal end portion (tip portion) of the balloon is connected to the distal end portion (tip portion) of the inner shaft, the outer shaft is a multilayer tube having three layers of an outer layer, an intermediate layer, and an inner layer, the intermediate layer includes a stainless steel rectangular wire, and the distal end portion (tip portion) of the inner shaft includes a stainless steel pipe.

In addition, the following balloon catheter has been reported in Patent Document 2. In the balloon catheter, a radiopaque marker is attached to a portion of the inner shaft to which an expansion effective portion of the balloon is projected, and has: a first contrast section located in the center of the expansion effective portion; a second contrast section indicating the distal end boundary of the expansion effective portion; and a third contrast section indicating the proximal end boundary of the expansion effective portion.

Patent Document 3 discloses that at a forward end chip 20, the tip tapered part 24 extending from the tip vicinity of a forward end balloon joint part 22 to the distal end 23 of a catheter is continuously reduced in wall thickness, the average wall thickness decrease gradient is set to 6-60 µm/mm, the length from the tip of the forward end balloon joint part 22 to the distal end 23 of the catheter is set to 3-10 mm, and the tube wall thickness of the distal end of the tip tapered part is set to 10-50 µm.

### PRIOR ART DOCUMENTS

### PATENT DOCUMENTS

[Patent Document 1] WO2019-156195
[Patent Document 2] JP 2016-174709 A
[Patent Document 3] JP2008264569

### SUMMARY OF THE INVENTION

### PROBLEMS TO BE SOLVED BY THE INVENTION

In the balloon catheter described in Patent Document 1, the stainless steel rectangular wire is included in the intermediate layer of the outer shaft, and the stainless steel pipe is included in the tip portion of the inner shaft, which allows to identify positions of the distal end portion and the proximal end portion of the balloon. However, positioning of the balloon to the desired treatment site is complicated. Furthermore, since the balloon extends longitudinally due to expansion of the balloon, depending on the position of the balloon in relation to the target treatment site, the treatment site may not be expandable with the balloon, and the balloon may need to be repositioned.

In regard to the balloon catheter described in Patent Document 2, a range and the center of the balloon expansion effective portion can be checked with the radiopaque marker provided on the inner shaft.

However, in a case where the structure disclosed in Patent Document 2 is applied to the balloon catheter, in which the balloon extends longitudinally, as described in Patent Document 1, that is, in a case where the radiopaque marker is attached onto the inner shaft, the balloon, the inner shaft, and the outer shaft in mutually different lengths are deformed simultaneously in the longitudinal direction unlike the balloon described in Patent Document 2. Thus, there is a problem that the position of the X-ray contrast portion indicating the center of the balloon effective expanded portion is significantly shifted from a relative position to the pre-expanded balloon.

As described above, in regard to the balloon catheter in which the balloon extends longitudinally during the expansion of the balloon, such a balloon catheter has not been disclosed that does not cause misalignment of the radiopaque marker due to a balloon expansion force.

### MEANS FOR SOLVING THE PROBLEMS

In order to solve the above problem, the present inventors intensively studied to discover the following inventions according to the appended claims.

The aforementioned objectives are achieved according to the invention by means of a balloon catheter according to claim 1. Preferred embodiments are defined in the dependent claims.

### EFFECT OF THE INVENTION

According to the present invention, the intermediate tube is fitted onto the inner shaft, and only the proximal end portion of the intermediate tube is fixed to the inner shaft. The radiopaque marker is fixed on the intermediate tube. Thus, the radiopaque marker remains in place without being affected by longitudinal extension of the inner shaft caused by expansion of the balloon. Therefore, the position of the radiopaque marker is not changed between the balloon contracted state and the balloon expanded state, which enables, in the balloon contracted state, accurate positioning of the balloon in the balloon expanded state to a target treatment site.

### BRIEF DESCRIPTION OF THE DRAWINGS

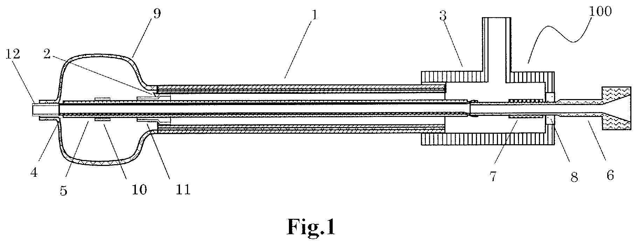

Fig. 1 is a schematic view of a balloon catheter in a longitudinal direction of the balloon catheter according to an embodiment of the present invention.

### MODE FOR CARRYING OUT THE INVENTION

A balloon catheter in the present invention includes: an outer shaft having flexibility; an inner shaft inserted into the outer shaft and having flexibility; an intermediate tube fitted onto the inner shaft, only a proximal end portion of the intermediate tube being fixed on the inner shaft; a longitudinally expandable balloon made of an elastic material and connected to a distal end portion of the outer shaft and a distal end portion of the inner shaft; and a radiopaque marker located inside the balloon and fixed on the intermediate tube.

A preferred embodiment of the present invention is described in detail below with reference to the drawing. However, the present invention is not limited to this mode. The same symbols are used for the same components, and thus the overlapping description is not made. In addition, proportions in the drawing do not necessarily correspond to those in the description.

Here, a "distal side" of the balloon catheter refers to a balloon side in a longitudinal direction of the balloon catheter. A "proximal end side" thereof refers to a holding member side in the longitudinal direction of the balloon catheter.

A "single-layer tube" refers to a tube structured to have a single-layer cross-sectional shape, while a "multilayer tube" refers to a tube made by combining multiple materials and structured to have a multilayer cross-sectional shape.

In regard to the outer shaft, the "distal end portion" of the outer shaft refers to an end portion on the balloon side in the longitudinal direction of the balloon catheter, and a "proximal end portion" of the outer shaft refers to an end portion on the holding member side in the longitudinal direction of the balloon catheter. In regard to the inner shaft, the "distal end portion" of the inner shaft refers to an end portion on the balloon side in the longitudinal direction of the balloon catheter, and a "proximal end portion" of the inner shaft refers to an end portion on the holding member side in the longitudinal direction of the balloon catheter. In regard to the intermediate tube, a "distal end portion" of the intermediate tube refers to an end portion on the balloon side in the longitudinal direction of the balloon catheter, and the "proximal end portion" of the intermediate tube refers to an end portion on the holding member side in the longitudinal direction of the balloon catheter.

In regard to the balloon, a "balloon contracted state" refers to a state where no external force is applied to the balloon, and a length from a distal end portion of the balloon to a proximal end portion of the balloon in the balloon contracted state refers to a "natural length". In regard to the balloon, a "balloon expanded state" refers to a state where fluid is supplied to the balloon and the balloon is expanded to a desired size required for treatment.

Fig. 1 is a schematic side view in the longitudinal direction of a balloon catheter according to the embodiment of the present invention. A balloon catheter 100 illustrated in Fig. 1 is formed of an outer shaft 1, an inner shaft 4, an intermediate tube 5, a radiopaque marker 10, a balloon 9, an anti-stretch member 2, a holding member 3, a push-in member 6, a pull-out prevention member 7, and a sealing member 8. An outer shaft assembly includes the outer shaft 1, the anti-stretch member 2, and the holding member 3, and an inner shaft assembly is formed of the inner shaft 4, the intermediate tube 5, the X-ray contrast marker 10, the push-in member 6, the pull-out prevention member 7, and the sealing member 8.

### <Outer shaft>

A structure of the outer shaft 1 can be either the single-layer tube or the multilayer tube. For example, in a case of the multilayer tube, a multilayer tube having an outer layer, an intermediate layer, and an inner layer is considered.

In regard to materials for the outer shaft 1, in a case where the outer shaft 1 is the multilayer tube having the three layers, a material for the outer layer is preferably a polymeric material having flexibility and excellent antithrombogenicity. Examples of such a polymeric material are vinyl chloride, polyurethanes, polyamides, polyether block amide copolymers, polypropylene, polyolefins, and polyethylene terephthalate. A polyurethane or a polyether block amide copolymer that matches a material for the balloon 9 is preferred to enable heat sealing with the balloon 9 described below. The intermediate layer only needs to be made of a metal wire, and an example thereof is stainless steel commonly used for medical equipment. In regard to the inner layer, a fluorine-based polymer such as PTFE can be used to improve slipperiness of the inner surface of the lumen of the outer shaft 1 and stretch resistance of the outer shaft 1. However, the material for the inner layer is not limited thereto.

When ISO 527 (Plastics - Determination of tensile properties, JIS K7161: Plastics - Determination of tensile properties) is applied to a test method for the outer shaft 1, strength of a central portion, which does not include an adhesive with another member, in the outer shaft 1 at longitudinal strain of 5% is 5.0 N to 15.0 N and, preferably, 5.5 N to 10.5 N. The length of the outer shaft 1 can be set appropriately according to the treatment target, but the length is preferably 200 mm to 1100 mm for cardiac valve stenosis.

### <Inner shaft>

The inner shaft 4 is a member, the inside of which serves as a lumen for a guidewire of the balloon catheter 100, and which forms an expansion lumen for the balloon 9 by being inserted into a lumen of the outer shaft 1.

When ISO 527 (Plastics - Determination of tensile properties, JIS K7161: Plastics - Determination of tensile properties) is applied to a test method for the inner shaft 4, strength of a central portion, which does not include the adhesive with another member, in the inner shaft 4 at longitudinal strain of 5% is 4.5 N to 14.5 N and preferably, 5.0 N to 10.0 N. Specific materials therefor include, but are not limited to, polyamides and polyether block amides.

In a relationship between the outer shaft 1 and the inner shaft 4, the strength of the outer shaft 1 at the longitudinal strain of 5% with respect to the strength of the inner shaft 4 at the longitudinal strain of 5% is 100% to 160%. The strength of the outer shaft being 160% or less at the time when the strength of the inner shaft is taken as 100% is preferred since it is possible to prevent shifting of the radiopaque marker, which is positioned in the balloon contracted state, to the distal side in the balloon expanded state. The strength of the outer shaft being 100% or greater at the time when the strength of the inner shaft is taken as 100% is also preferred since it is possible to prevent shifting of the radiopaque marker, which is positioned in the balloon contracted state, to the proximal end side in the balloon expanded state.

### <Intermediate tube>

The intermediate tube 5 is a member that prevents kinking or buckling of the inner shaft 4, which is caused by a restoring force of the balloon 9 when the balloon 9 is stretched for the purpose of reducing bulkiness of the balloon 9 for insertion of the balloon catheter 100 into a blood vessel. The inner shaft 4 and the intermediate tube 5 are mutually slidable since the intermediate tube 5 is fitted onto the inner shaft 4 over almost the entire length thereof, except for a part of the tip portion of the inner shaft 4. Thus, such a mechanism is realized that stretching action only occurs to the inner shaft 4 due to a tensile force applied to the inner shaft 4 during expansion of the balloon. This relationship between the inner shaft 4 and the intermediate tube 5 enables simultaneous achievement of buckling resistance during stretching of the balloon and flexibility during the expansion of the balloon.

The inner shaft assembly is formed by fitting the intermediate tube 5 onto the inner shaft 4 and fixing only a proximal end portion of the intermediate tube 5 to the inner shaft 4. When only the proximal end portion of the intermediate tube 5 is fixed on the proximal end portion of the inner tube shaft 4, just as described, it is possible to maintain such a mechanism that the stretching action only occurs to the inner shaft 4 while preventing interference of the members, such as moving over the other member, with minimum fixed portions.

In a case of a resin, materials for the intermediate tube 5 include, but are not limited to, polyimides, polyether ether ketones, polyphenylene sulfide, polyetherimides, and polyamideimides. A polyimide is more preferred from the viewpoint of wear resistance and chemical stability.

When ISO 527 (Plastics - Determination of tensile properties, JIS K7161: Plastics - Determination of tensile properties) is applied to a test method for the intermediate tube 5, the tensile modulus thereof is 1.3 GPa to 4 GPa, and the thickness thereof is preferably 0.10 mm to 0.30 mm. The tensile modulus of 1.3 GPa or greater is preferred since a possibility of wrinkling can be reduced at the time when the intermediate tube 5 is bent during an operation of the catheter. The tensile modulus of 4 GPa or less is preferred since appropriate flexibility is provided. In addition, a thickness of 0.10 mm or greater is preferred since the possibility of wrinkling can be reduced at the time when the intermediate tube 5 is bent during the operation of the catheter. The thickness of 0.30 mm or less is preferred since the appropriate flexibility is provided.

A clearance between the inner diameter of the intermediate tube 5 and the outer diameter of the inner shaft 4 is preferably 0.01 mm to 0.2 mm. The clearance of 0.01 mm or greater is preferred since the slidability between the intermediate tube 5 and the inner shaft 4 falls within an appropriate range. The clearance of 0.2 mm or less is preferred since the radiopaque marker fixed on the intermediate tube is stabilized.

### <Radiopaque marker>

The radiopaque marker 10 is a member that allows an operator to know the position of the balloon 9 when the balloon catheter 100 is inserted into a body. The radiopaque marker 10 is fixed on the surface of the intermediate tube 5. Fixing methods to the intermediate tube 5 include, but are not limited to, caulking, adhesion, and embedding. The radiopaque marker 10 is preferably fixed in a thickness of 40 µm or greater over an entire circumference of the intermediate tube, but is not limited thereto.

In the balloon contracted state, the center of the radiopaque marker 10 is preferably located within a range of 5.0 mm to 8.5 mm from the distal end portion of the outer shaft. When the center of the radiopaque marker is located 5.0 mm to 8.5 mm therefrom, it is possible to reduce shifting of the relative position of the center of the balloon to the radiopaque marker between the balloon contracted state and the balloon expanded state.

When the longitudinal length of the balloon in the balloon contracted state is taken as 100%, the center of the radiopaque marker is preferably located 10% to 80% from the proximal end portion of the balloon and, more preferably, located 30% to 60% therefrom. When the center of the radiopaque marker is located 10% to 80% from the proximal end portion of the balloon, it is possible to reduce shifting of the relative position of the center of the balloon to the radiopaque marker between the balloon contracted state and the balloon expanded state. When the center of the radiopaque marker is located 30% to 60% from the proximal end portion of the balloon, the shifting of the relative position can be reduced to the extent that it is difficult to visually confirm the shifting.

Any radiopaque material can be used for the radiopaque marker, and an example of the radiopaque material is a heavy metal compound that is radiopaque and stable for a long period. Specific examples thereof include a metal material containing at least one type of a tungsten compound, a bismuth compound, barium sulfate, palladium, platinum, gold, silver, and tantalum. From the viewpoint of corrosion resistance and hardness, a platinum-iridium alloy is preferred.

### <Push-in member>

The push-in member 6 is a member used by the operator to stretch the balloon 9 in order to insert the balloon catheter 100 into a blood vessel. The push-in member 6 has at least two pipe portions with different outer diameters at a location other than a holding portion. The outer diameters of the pipe portions are gradually increased from the distal end side toward the proximal end side, and an outer-diameter transition portion of each of them is tapered. In addition, a distal end portion of the push-in member 6 is connected to the proximal end portion of the inner shaft 4.

<Balloon>

In the balloon catheter 100, the balloon 9 is formed of an elastic material. Specific examples of the elastic material forming the balloon 9 include silicone, polyether block amide copolymers, polyurethanes, natural rubber, and synthetic rubber. The balloon 9 may have a multilayer structure. In a case of the balloon having the multilayer structure, for example, the balloon may be obtained by bonding a mesh and natural rubber with rubber cement. The mesh is formed by knitting false twisted yarns made of a polyurethane or a polyester in a cylindrical shape. Hardness of the balloon 9 may vary according to the treatment target. However, when the balloon 9 is formed of a single material, Shore A hardness of the material is preferably 100 or less.

In a case where the elastic material forming the balloon 9 is a material, such as natural rubber or synthetic rubber, that is difficult to be sealed, it is normally difficult to attach such a balloon 9 to the outer shaft 1. In this case, a short pipe made of a hard resin or metal, for example, may be inserted into the tip portion of the outer shaft 1 in a manner to be projected from the tip portion of the outer shaft 1. Then, the material that is difficult to be sealed may be fixed on a projected portion of the pipe by winding a thread such as a nylon string around the material.

Similarly, in a case where the elastic material forming the balloon 9 is the material, such as natural rubber or synthetic rubber, that is difficult to be sealed, it is also normally difficult to attach the balloon 9 to the inner shaft 4. In this case, a short pipe made of the hard resin or the metal, for example, may be inserted into the tip portion of the inner shaft 4. Then, the material that is difficult to be sealed may be fixed on an outer periphery of a portion of the inner shaft 4 where the pipe is present by winding the thread such as the nylon string around the material.

The natural length of the balloon 9 can be set appropriately according to the treatment target, and the length is preferably 20 mm to 30 mm for cardiac valve stenosis.

The diameter of the balloon 9, which is orthogonal to the longitudinal direction, during the expansion of the balloon 9 can be set appropriately according to the treatment target, and is preferably 13 mm to 30 mm for cardiac valve stenosis.

### <Anti-stretch member>

The anti-stretch member 2 is a member that prevents stretching of the outer shaft 1 caused by a restoring force of the balloon 9 to return to the natural length thereof when the balloon catheter 100 is inserted into the blood vessel with the balloon 9 being deformed. In order to achieve this, the anti-stretch member 2 is formed of a material with a greater elastic stress than the restoring force of the balloon 9. In addition, the anti-stretch member 2 can have any shape that prevents stretching of the outer shaft 1, and the anti-stretch member 2 in a monofilament, multifilament, or strip shape is considered, for example. This anti-stretch member 2 is stretched or bonded to the inside of the outer shaft 1 for the entire longitudinal length.

In the balloon catheter 100, the longitudinal length of the monofilament anti-stretch member 2 is longer than the longitudinal length of the outer shaft 1. Thus, at each of ends that are an opening on the distal side and an opening on the proximal end side of the lumen of the outer shaft 1, an end of the anti-stretch member 2 is folded back onto the outer surface of the outer shaft 1.

As a material for the anti-stretch member 2, a material that has high tensile strength and does not inhibit the balloon catheter from following a curve of the blood vessel or the like is preferred, and aramid fiber or polyacrylate fiber is preferred.

### <Sealing member>

The sealing member 8 that allows the inner shaft 4 and the outer shaft 1 to slide in a liquid-tight state is provided to the opening on the proximal end side of the outer shaft 1, and the holding member 3 held by the operator during the operation is attached in a manner to surround the outer periphery of the outer shaft 1.

### <Holding member>

The holding member 3 is a member held by the operator and can have an ergonomic shape to facilitate the operation thereof. An example of the shape is a Y-shape but is not limited thereto. The holding member 3 is attached to the proximal end side of the outer shaft 1 in a manner to surround the outer periphery thereof.

From the viewpoint of ensuring moldability and strength, a material for the holding member 3 is preferably a resin with certain hardness. Examples of such a material are plastics including a styrene resin, an acrylic resin, polypropylene, polyethylene, a fluorine resin, and polyacetal.

### <Push-in member>

As a material for a pipe portion of the push-in member 6, a hard resin or a metal material is preferably used to allow the operator to easily push in the push-in member 6, and the metal material is preferably used. In addition, as the metal material, stainless steel is preferred from the viewpoint of corrosion resistance. A handle portion is preferably provided in a proximal end portion of the push-in member 6 to ensure a firm grip by the operator, and a material for this handle portion is preferably a hard resin or a metal material. Furthermore, from the viewpoint of slip prevention, a surface of the handle portion is preferably knurled or sandblasted to have a rough shape.

In regard to a step in the pipe portion of the push-in member 6, the pipe portion on the proximal end side has a step of 0.3 mm to 0.4 mm and a taper transition length of 0.5 mm to 1 mm, which, however, depend on a tightening force of the sealing member 8. In this way, the operator can feel the step portion with tactile sensation when sliding the inner shaft assembly and the outer shaft assembly with respect to each other. In this case, since a load on the sealing member 8 described below at the time of moving over the step in the pipe portion of the push-in member 6 is 10 N to 15 N, the operation for moving over the step can be performed without stress.

### <Pull-out prevention member>

The pull-out prevention member 7 is a member having a cylindrical shape with a thickness of 0.1 mm to 0.4 mm, connected onto a pipe with the second largest outer diameter in the pipe portion of the push-in member 6, and preventing a reduction in the natural length of the balloon 9 on the proximal end side in the balloon catheter 100.

The material for the pull-out prevention member 7 is preferably a hard resin or metal. When the pull-out prevention member 7 is attached to the push-in member 6, an attachment method such as adhesion using the adhesive, welding, or sealing may be selected according to the material for the pull-out prevention member 7.

### <Sealing member>

The sealing member 8 enables the inner shaft assembly described below to slide with respect to the outer shaft assembly while keeping the inside of the balloon catheter 100 in the liquid tight state by sealing the opening in the holding member 3.

As a material for the sealing member 8, a soft material is preferred from the viewpoint of allowing sliding of the inner shaft assembly while maintaining liquid tightness. For example, silicone rubber, synthetic rubber, or a styrene thermoplastic elastomer is preferred.

The seal member 8 may be structured such that, for example, a slit valve formed by making a slit in a part of a soft material sheet is incorporated as the sealing member 8 into the holding member 3 or that a cap fitting structure is provided to the holding member 3, so as to tighten the sealing member 8 formed of an O-ring or cylinder shaped soft material.

### <Assembling outer shaft assembly, inner shaft assembly, and balloon>

The balloon catheter 100 is formed by inserting the inner shaft assembly described above into the outer shaft assembly and bonding the inner shaft assembly and the distal end portion of the outer shaft assembly to the balloon 9.

When the balloon catheter 100 is formed, the inner shaft assembly is inserted into the outer shaft assembly such that the sealing member 8 in the outer shaft assembly is located at a position of a proximal end portion of a small-diameter portion of the push-in member 6 in the inner shaft assembly. **In** this state, the pull-out prevention member 7 is attached onto the small-diameter portion of the push-in member 6 while avoiding the sealing member 8. Then, the balloon 9 is connected to the distal end portion of the outer shaft 1 and the distal end portion of the inner shaft 4 (a portion onto which the intermediate tube 5 is not fitted), and this state corresponds to the natural length state of the balloon 9. In this way, the balloon catheter 100 is formed.

In order to further increase rigidity of the tube portion of the inner shaft assembly at the time of forming the balloon catheter 100, the pipe portion of the push-in member 6 is preferably advanced into the lumen of the outer shaft 1. In this case, a length thereof may be set appropriately according to the treatment target by the balloon catheter. For an approach from a femoral artery to a cardiac valve, 600 mm to 900 mm is preferred. For an approach from a femoral vein to the left atrium, 500 mm to 800 mm is preferred.

In addition, when the pipe portion of the push-in member 6 is advanced into the outer shaft 1, lengths of the inner shaft 4 and the intermediate tube 5 are adjusted appropriately according to the length of the balloon catheter. The length of the inner shaft 4 is preferably 200 mm to 400 mm for the approach from the femoral artery to the cardiac valve, and is preferably 100 mm to 300 mm for the approach from the femoral vein to the left atrium.

### <Method for measuring strength and tensile modulus at longitudinal strain of 5%>

The evaluation was performed using a TENSELON universal material testing instrument (A&D Company, Limited, model: RTG-1250) equipped with a 1 kN load cell with reference to ISO 10555-1 (Intravascular catheters - Sterile and single-use catheters -).

More specifically, test pieces (the intermediate tube, the outer shaft, and the inner shaft) were each cut out to have a length of 100 mm in a measurement direction and immersed in purified water controlled at 37°C ± 2°C for 24 hours. Then, within 5 minutes after removal of the test pieces from purified water, the TENSILON universal material testing instrument (A&D Company, Limited, model: RTG-1250) was used to stretch the test pieces having an original length (distance between chucks) of 20 mm at a tensile speed of 400 mm/minute for measurement under environment of a room temperature at 25°C ± 3°C and 55% RH to 65% RH. The original length was taken as 100%, and the strength (N) at the time when each of the test pieces reached 105% was read as the strength at the longitudinal strain of 5%.

### EXAMPLES

Specific examples of the balloon catheter in the present invention are described below with reference to Fig. 1.

### (Example 1)

A tube having a three-layer structure was molded by using the polyether block amide copolymer as the material for the outer layer, using stainless-steel rectangular wires as the material for the intermediate layer having a braided structure, and using PTFE as the material for the inner layer. A single-layer tube (4 mm in length) formed of the polyether block amide copolymer was attached to a tip portion of this tube with the three-layer structure by heat sealing to prepare a braid tube. This braid tube had an outer diameter of 3.1 mm and an inner diameter of 2.6 mm.

Next, a single-stepped pipe 11 having a small-diameter portion on the distal end side and a large-diameter portion on the proximal end side (the small-diameter portion had an outer diameter of 2 mm, an inner diameter of 1.84 mm, and a length of 7 mm, the large-diameter portion had an outer diameter of 2.4 mm, an inner diameter of 2.24 mm, and a length of 3 mm, and the pipe 11 was made of stainless steel) was prepared. An end of the aramid fiber (having a length of 1200 mm and a diameter of 0.3 mm) was wound around and fixed on a step of the single-stepped pipe 11. After the aramid fiber passed through the braid tube, the large-diameter portion of the single-stepped pipe 11 and a tip portion of the braid tube were fixed on each other with an adhesive to prepare the outer shaft 1. Five tubes, each of which had a length of 100 mm, were cut out from a central portion of the braid tube, which did not include the adhesive, in the outer shaft 1 and used as the test pieces to evaluate a mechanical property. An average value of the strength at the longitudinal strain of 5% was 8.5 N.

Next, a Y-shaped connector having a cap fitting structure, to which an O-ring could be fitted, was used as the holding member 3. As the anti-stretch member 2, the aramid fiber was stretched in a manner to serve as the anti-stretch member 2 over the entire length of the lumen of the outer shaft 1. Then, in a state where the aramid fiber was folded back onto the outer periphery of the proximal end portion of the outer shaft 1, the proximal end portion of the outer shaft 1 and a tube connection port of the Y-shaped connector were fixed on each other with the adhesive.

Next, a stainless-steel pipe having a handle portion and three-stepped outer diameters was prepared as the push-in member 6. When portions having different diameters in the push-in member 6 were respectively defined as a large-diameter portion, an intermediate portion, and a small-diameter portion, in the order from the proximal end side in the longitudinal direction, the large-diameter portion had an outer diameter of 2.1 mm and a length of 60 mm, the intermediate portion had an outer diameter of 1.8 mm and a length of 10 mm, and a stainless steel pipe having an outer diameter of 2.0 mm was fitted onto the intermediate portion and welded to the push-in member 6. The length of a tapered portion shifting from the large-diameter portion to the intermediate portion was 0.5 mm. The small-diameter portion had an outer diameter of 1.16 mm and a length of 805 mm. A minimum inner diameter of the push-in member 6 was 1.0 mm.

Next, a screw-type cap for the holding member 3 and an O-ring having an inner diameter of 1.4 mm and a wire diameter of 1.5 mm were fitted onto the push-in member 6. Then, the cap was arranged to the large-diameter portion of the push-in member 6, and the O-ring was arranged to a position between the proximal end side of the stainless steel pipe, which was welded to the intermediate portion of the push-in member 6, and the tapered portion from the large-diameter portion.

The inner shaft 4 was prepared as follows. A proximal end portion of a polyamide tube (an outer diameter of 1.3 mm and an inner diameter of 0.94 mm) was enlarged and fixed on a distal end portion of the small-diameter portion of the push-in member 6 with the adhesive. Furthermore, a distal end of this tube was enlarged to fit and fix a catheter tip stainless steel pipe 12 (an outer diameter of 1.16 mm, an inner diameter of 1.0 mm, and a length of 7 mm) to a lumen of the tube with the adhesive. Five tubes, each of which had a length of 100 mm, were cut out from a central portion of the polyamide tube, which did not include the adhesive, in the inner shaft 4 and used as test pieces to evaluate the mechanical property (a test method: ISO 527). An average value of the strength at the longitudinal strain of 5% was 6.5 N. The strength of the outer shaft 1 at the longitudinal strain of 5% with respect to the strength of the inner shaft at the longitudinal strain of 5% was 130%.

As the intermediate tube 5, a polyimide tube (an inner diameter of 1.35 mm and an outer diameter of 1.47 mm) having a tensile modulus of 3.4 GPa (the test method: ASTM D790) was used. Before the catheter tip stainless steel pipe 12 attached to the distal end of the inner shaft 4 was bonded, this intermediate tube 5 was fitted onto the tube constituting the inner shaft 4, so as to bring the proximal end portion of the intermediate tube 5 into contact with the tip of the small-diameter portion of the pipe in the push-in member 6. Then, only about 2 mm of the proximal end portion of the intermediate tube 5 was fixed on the inner shaft 4 with the adhesive. Five tubes, each of which had a length of 100 mm, were cut out from a central portion of the intermediate tube 5, which did not include the adhesive, and used as test pieces to evaluate the mechanical property. Then, an average value thereof was used. A clearance between an inner diameter of the intermediate tube and an outer diameter of the inner shaft was 0.06 mm.

The single radiopaque marker 10 was fitted and fixed on the intermediate tube 5 with the adhesive such that a center of the radiopaque marker was located 11.5 mm from the proximal end portion of the balloon. The radiopaque marker 10 made of a platinum-iridium alloy had a cylindrical shape with an outer diameter of 1.60 mm, an inner diameter of 1.52 mm, and a length of 4 mm. Here, the radiopaque marker 10 was fitted onto the intermediate tube 5 before the catheter tip stainless steel pipe 12, which was connected to the distal end of the inner shaft 4, was bonded.

The inner shaft assembly including the inner shaft 4, the tube 5, the push-in member 6, and the radiopaque marker 10 was inserted into the outer shaft assembly. The cap for the holding member 3 was fitted to the holding member 3, and the O-ring serving as the sealing member 8 was tightened such that 15 N is exerted when the O-ring moved from the intermediate portion onto the large-diameter portion of the push-in member 6 (synonymous with a sliding force between the inner shaft assembly and the outer shaft assembly). Then, the O-ring was adjusted to be located at the proximal end of the intermediate portion of the push-in member 6, and this state was taken as a natural length state of the balloon in the balloon catheter 100.

In the balloon catheter 100, the balloon 9 was formed to have a three-layer structure. An inner-layer balloon made of natural rubber and having an inner diameter of 4.0 mm and a film thickness of 0.3 mm on one side was connected onto each of the small-diameter portion of the stepped pipe in the outer shaft 1 and the catheter tip stainless steel pipe 12 of the inner shaft 4 by winding a No. 0.2 nylon string and then fixed thereto with the adhesive. Then, this was used as an inner balloon. Furthermore, an outer balloon assembly was arranged thereon. The outer balloon assembly was formed by bonding natural rubber, which had an inner diameter of 4.0 mm and a film thickness of 0.3 mm on one side, and the mesh, which was formed by knitting the false twisted yarns made of the polyurethane or the polyester in the cylindrical shape, with the rubber cement. The outer balloon assembly was connected onto each of the small-diameter portion of the single-stepped pipe of the outer shaft 1 and the catheter tip stainless steel pipe 12 of the inner shaft 4 by winding a No. 0.6 nylon string and then fixed thereto with the adhesive. Then, this was used as an outer balloon.

In this way, the balloon 9 with the three-layer structure having the inner layer made of the natural rubber, the intermediate layer made of the mesh, and the outer layer made of the natural rubber was obtained. **In** addition, the natural length of the balloon in the longitudinal direction of the catheter was set to 25 mm, and a balloon diameter during the expansion was set to 22 mm. When the length of 25 mm of the balloon in the catheter longitudinal direction in the balloon contracted state was taken as 100%, the distance between the center of the radiopaque marker and the proximal end portion of the balloon was 11.5 mm, and the center of the radiopaque marker was located 46% from the proximal end portion of the balloon.

### (Example 2)

The braid tube with the strength of 8.5 N at the strain of 5% of the outer shaft in Example 1 was replaced with a braid tube with strength of 10.0 N at the strain of 5% of the outer shaft. The polyamide tube with the strength of 6.5 N at the strain of 5% of the inner shaft in Example 1 was replaced with a polyamide tube with strength of 10.0 N at the strain of 5% of the outer shaft. **In** addition, when the length of 25 mm of the balloon in the catheter longitudinal direction in the balloon contracted state was taken as 100%, the center of the radiopaque marker was changed to be located 30% from the proximal end portion of the balloon. Furthermore, as the intermediate tube, a tube made of polyvinyl chloride (PVC) and having the tensile modulus of 1.3 GPa (the test method: ASTM D790) was used, and the clearance was changed to 0.20 mm to prepare Example 2.

### (Example 3)

The braid tube with the strength of 8.5 N at the strain of 5% of the outer shaft in Example 1 was replaced with a braid tube with strength of 14.0 N at the strain of 5% of the outer shaft. The polyamide tube with the strength of 6.5 N at the strain of 5% of the inner shaft in Example 1 was replaced with a polyamide tube with strength of 13.0 N at the strain of 5% of the outer shaft. **In** addition, when the length of 25 mm of the balloon in the catheter longitudinal direction in the balloon contracted state was taken as 100%, the center of the radiopaque marker was changed to be located 60% from the proximal end portion of the balloon. Furthermore, as the intermediate tube, a tube made of a polyether ether ketone (PEEK) and having the tensile modulus of 4 GPa (the test method: ASTM D790) was used, and the clearance was changed to 0.01 mm to prepare Example 3.

### (Example 4)

The braid tube with the strength of 8.5 N at the strain of 5% of the outer shaft in Example 1 was replaced with a braid tube with strength of 7.0 N at the strain of 5% of the outer shaft. The polyamide tube with the strength of 6.5 N at the strain of 5% of the inner shaft in Example 1 was replaced with a polyamide tube with strength of 10.0 N at the strain of 5% of the outer shaft. In addition, when the length of 25 mm of the balloon in the catheter longitudinal direction in the balloon contracted state was taken as 100%, the center of the radiopaque marker was changed to be located 10% from the proximal end portion of the balloon. Furthermore, as the intermediate tube, a tube made of polyethylene (PE) and having the tensile modulus of 0.5 GPa (the test method: ASTM D790) was used, and the clearance was changed to 0.8 mm to prepare Example 4.

### (Example 5)

The braid tube with the strength of 8.5 N at the strain of 5% of the outer shaft in Example 1 was replaced with a braid tube with strength of 15.0 N at the strain of 5% of the outer shaft. The polyamide tube with the strength of 6.5 N at the strain of 5% of the inner shaft in Example 1 was replaced with a polyamide tube with strength of 9.5 N at the strain of 5% of the outer shaft. In addition, when the length of 25 mm of the balloon in the catheter longitudinal direction in the balloon contracted state was taken as 100%, the center of the radiopaque marker was changed to be located 80% from the proximal end portion of the balloon. Furthermore, as the intermediate tube, a tube made of carbon fiber reinforced plastic (CFRP) and having the tensile modulus of 8 GPa (the test method: ASTM D790) was used, and the clearance was changed to 0.005 mm to prepare Example 4.

### (Example 6)

The braid tube with the strength of 8.5 N at the strain of 5% of the outer shaft in Example 1 was replaced with a braid tube with strength of 5.5 N at the strain of 5% of the outer shaft. The polyamide tube with the strength of 6.5 N at the strain of 5% of the inner shaft in Example 1 was replaced with a polyamide tube with strength of 4.5 N at the strain of 5% of the outer shaft. In addition, when the length of 25 mm of the balloon in the catheter longitudinal direction in the balloon contracted state was taken as 100%, the center of the radiopaque marker was changed to be located 60% from the proximal end portion of the balloon. Furthermore, as the intermediate tube, a tube made of a polyacetal resin (POM) and having the tensile modulus of 2.5 GPa (the test method: ASTM D790) was used, and the clearance was changed to 0.10 mm to prepare the catheter of Example 4.

### (Comparative Example 1)

Comparative Example 1 was prepared in the same manner as Example 1, except that, as a joint position of the intermediate tube, only the distal end of the intermediate tube was fixed to the inner shaft.

### (Comparative Example 2)

Comparative Example 2 was prepared in the same manner as Example 1, except that the intermediate tube was not used and the radiopaque marker was directly fixed, with the adhesive, onto a portion of the inner shaft located 7.5 mm from the distal end portion of the outer shaft.

### (Evaluation method of position of radiopaque marker)

A fluoroscopic X-ray image was captured in the balloon contracted state. Then, a length (L₁) from the distal end portion of the single-stepped pipe 11, which was connected to the tip of the outer shaft, to the proximal end portion of the catheter tip stainless steel pipe 12, which was connected to the tip of the inner shaft, during the contraction of the balloon and a length (L₂) from the distal end portion of the single-stepped pipe 11, which was connected to the tip of the outer shaft, to the center of the radiopaque marker 10 in the balloon contracted state were measured with a ruler.

Next, the balloon was expanded using a contrast agent diluted fivefold with a saline solution, and the fluoroscopic X-ray image was captured. Then, a length (L₁') from the distal end portion of the single-stepped pipe 11, which was connected to the tip of the outer shaft, to the proximal end portion of the catheter tip stainless steel pipe 12, which was connected to the tip of the inner shaft, in the balloon expanded state and a length (L₂') from the distal end portion of the single-stepped pipe 11, which was connected to the tip of the outer shaft, to the center of the radiopaque marker 10 in the balloon expanded state were measured with the ruler.

### (Determination method)

A change rate of the balloon expanded state from the balloon contracted state was calculated using following Equation 1 on the basis of the lengths (L₁, L₁') from the distal end portion of the single-stepped pipe 11, which was connected to the tip of the outer shaft, to the proximal end portion of the catheter tip stainless steel pipe 12, which was attached to the tip of the inner shaft, in the balloon contracted state and the balloon expanded state and the lengths (L₂, L₂') from the distal end portion of the single-stepped pipe 11, which was connected to the tip of the outer shaft, to the center of the radiopaque marker 10 in the balloon contracted state and the balloon expanded state. $\left(\left({L}_{1}\times {L}_{2}′/\left({L}_{1}′\times {L}_{2}\right)-1\right)\times 100\right) \left(%\right)$ Equation 1

When the change rate obtained from Equation 1 fell within ±20%, it was determined to be acceptable (in particular, classified as excellent within ±10% and other percentages as good). Otherwise, it was determined to be unacceptable (poor), and the results were listed in Table 1 and Table 2. When the change rate falls within ±20%, the balloon can be positioned easily during surgery. The change rate that falls within ±10% promises easy handling even for a novice.

**[Table 1]**

| Item | Example 1 | Example 2 | Example 3 | Example 4 |
|---|---|---|---|---|
| Intermediate tube | Present | Present | Present | Present |
| Joint position of intermediate tube | Only the proximal end side of the intermediate tube is fixed to the inner shaft. | Only the proximal end side of the intermediate tube is fixed to the inner shaft. | Only the proximal end side of the intermediate tube is fixed to the inner shaft. | Only the proximal end side of the intermediate tube is fixed to the inner shaft. |
| Strength (N) of outer shaft at 5% strain | 8.5 | 10.0 | 14.0 | 7.0 |
| Strength (N) of inner shaft at 5% strain | 6.5 | 10.0 | 13.0 | 10.0 |
| Strength rate (%) between inner and outer shafts | 131 | 100 | 107 | 70 |
| Marker position (%) | 46 | 30 | 60 | 10 |
| Tensile modulus (GPa) of intermediate tube | 3.4 | 1.3 | 4 | 0.5 |
| Clearance (mm) | 0.06 | 0.20 | 0.01 | 0.80 |
| Evaluation (position accuracy) | Excellent | Excellent | Excellent | Good |

**[Table 2]**

| Item | Example 5 | Example 6 | Comparative Example 1 | Comparative Example 2 |
|---|---|---|---|---|
| Intermediate tube | Present | Present | Present | Absent |
| Joint position of intermediate tube | Only the proximal end side of the intermediate tube is fixed to the inner shaft. | Only the proximal end side of the intermediate tube is fixed to the inner shaft. | Only the proximal end side of the intermediate tube is fixed to the inner shaft. | - |
| Strength (N) of outer shaft at 5% strain | 15.0 | 5.5 | 8.5 | 8.5 |
| Strength (N) of inner shaft at 5% strain | 9.5 | 4.5 | 6.5 | 6.5 |
| Strength rate (%) between inner and outer shafts | 157 | 122 | 131 | 131 |
| Marker position (%) | 80 | 60 | 46 | 46 |
| Tensile modulus (GPa) of intermediate tube | 8 | 2.5 | 3.4 | - |
| Clearance (mm) | 0.005 | 0.10 | 0.06 | 0.06 |
| Evaluation (position accuracy) | Good | Excellent | Poor | Poor |

### (Evaluation Results)

As shown in Table 1 and Table 2, compared to Comparative Examples 1, 2, in Example 1 to Example 4, the change in the relative position between the balloon and the radiopaque marker was small between the balloon contracted state and the balloon expanded state, indicating superior catheter operability.

### INDUSTRIAL APPLICABILITY

The present invention can be used favorably for a medical balloon catheter used inside the body, such as a balloon catheter used for endovascular treatment including valve stenosis treatment.

### DESCRIPTION OF SYMBOLS

1: outer shaft, 2: anti-stretch member, 3: holding member, 4: inner shaft, 5: intermediate tube, 6: push-in member, 7: pull-out prevention member, 8: sealing member, 9: balloon, 10: radiopaque marker, 11: single-stepped pipe, 12: catheter tip stainless steel pipe, 100: balloon catheter

## Claims

1. A balloon catheter (100) comprising:
an outer shaft (1) having flexibility;
an inner shaft (4) inserted into the outer shaft (1) and having flexibility;
an intermediate tube (5) wherein the inner shaft (4) and the intermediate tube (5) are mutually slidable and wherein the intermediate tube (5) is fitted onto the inner shaft (1) over almost the entire length thereof except for a part of the tip potion of the inner shaft (4) and wherein only the proximal end portion of the intermediate tube (5) is fixed on the proximal end portion of the inner shaft (4);
a longitudinally expandable balloon (9) made of an elastic material and connected to a distal end portion of the outer shaft and a distal end portion of the inner shaft; and
a radiopaque marker (10) located inside the balloon (9) and fixed on the intermediate tube (5),
wherein strength of the inner shaft (4) and strength of the outer shaft (1) at longitudinal strain of 5% are 4.5 to 14.5 N and 5.0 to 15.0 N, respectively, and
the strength of the outer shaft (1) at the longitudinal strain of 5% with respect to the strength of the inner shaft (4) at the longitudinal strain of 5% is 100% to 160%,
wherein a tensile modulus of the intermediate tube (5) is 1.3 to 4 GPa.

2. The balloon catheter of claim 1, wherein a clearance between an inner diameter of the intermediate tube (5) and an outer diameter of the inner shaft (4) is 0.01 to 0.20 mm.

3. The balloon catheter of claim 1 or 2, wherein, when a longitudinal length of the balloon in a balloon contracted state is taken as 100%, a center of the radiopaque marker is located 30 to 60% from a proximal end portion of the balloon.

## Patentansprüche

1. Ballonkatheter (100), umfassend:
einen äußeren Schaft (1), der Flexibilität aufweist;
einen inneren Schaft (4), der in den äußeren Schaft (1) eingesetzt ist und Flexibilität aufweist;
ein Zwischenrohr (5), wobei der innere Schaft (4) und das Zwischenrohr (5) gegenseitig verschiebbar sind und wobei das Zwischenrohr (5) auf den inneren Schaft (1) über nahezu dessen gesamte Länge mit Ausnahme eines Teils des Spitzenabschnitts des inneren Schafts (4) aufschoben ist und wobei nur der proximale Endabschnitt des Zwischenrohrs (5) an dem proximalen Endabschnitt des inneren Schafts (4) fixiert ist;
einen in Längsrichtung expandierbaren Ballon (9), der aus einem elastischen Material hergestellt und mit einem distalen Endabschnitt des äußeren Schafts und einem distalen Endabschnitt des inneren Schafts verbunden ist; und
einen röntgenopaken Marker (10), der innerhalb des Ballons (9) angeordnet und an dem Zwischenrohr (5) fixiert ist,
wobei die Festigkeit des inneren Schafts (4) und die Festigkeit des äußeren Schafts (1) bei einer Längsdehnung von 5 % 4,5 bis 14,5 N bzw. 5,0 bis 15,0 N betragen, und
die Festigkeit des äußeren Schafts (1) bei der Längsdehnung von 5 % in Bezug auf die Festigkeit des inneren Schafts (4) bei der Längsdehnung von 5 % 100 % bis 160 % beträgt, wobei ein Zugmodul des Zwischenrohrs (5) 1,3 bis 4 GPa beträgt.

2. Ballonkatheter nach Anspruch 1, wobei ein Spiel zwischen einem Innendurchmesser des Zwischenrohrs (5) und einem Außendurchmesser des inneren Schafts (4) 0,01 bis 0,20 mm beträgt.

3. Ballonkatheter nach Anspruch 1 oder 2, wobei, wenn eine Länge des Ballons in Längsrichtung in einem Zustand, in dem der Ballon kontrahiert ist, als 100 % angenommen wird, ein Zentrum des röntgenopaken Markers 30 bis 60 % von einem proximalen Endabschnitt des Ballons entfernt angeordnet ist.

## Revendications

1. Cathéter à ballonnet (100) comprenant :
une tige extérieure (1) ayant une flexibilité ;
une tige intérieure (4) insérée dans la tige extérieure (1) et ayant une flexibilité ;
un tube intermédiaire (5), dans lequel la tige intérieure (4) et le tube intermédiaire (5) peuvent coulisser l'un par rapport à l'autre, dans lequel le tube intermédiaire (5) est monté sur la tige intérieure (1) sur la quasi-totalité de sa longueur, à l'exception d'une partie de la portion d'extrémité distale de la tige intérieure (4), et dans lequel seule la portion d'extrémité proximale du tube intermédiaire (5) est fixée sur la portion d'extrémité proximale de la tige intérieure (4) ;
un ballonnet (9) extensible longitudinalement, constitué d'un matériau élastique et relié à une portion d'extrémité distale de la tige extérieure et à une portion d'extrémité distale de la tige intérieure ;
un marqueur radio-opaque (10) situé à l'intérieur du ballonnet (9) et fixé sur le tube intermédiaire (5),
dans lequel la résistance de la tige intérieure (4) et la résistance de la tige extérieure (1) à une déformation longitudinale de 5 % sont respectivement de 4,5 à 14,5 N et de 5,0 à 15,0 N, et
la résistance de la tige extérieure (1) à la déformation longitudinale de 5 % par rapport à la résistance de la tige intérieure (4) à la déformation longitudinale de 5 % est de 100 % à 160 %, dans lequel le module de traction du tube intermédiaire (5) est de 1,3 à 4 GPa.

2. Cathéter à ballonnet selon la revendication 1, dans lequel un jeu entre un diamètre intérieur du tube intermédiaire (5) et un diamètre extérieur de la tige intérieure (4) est de 0,01 à 0,20 mm.

3. Cathéter à ballonnet selon la revendication 1 ou 2, dans lequel, lorsque la longueur longitudinale du ballonnet à l'état contracté est prise comme étant de 100 %, un centre du marqueur radio-opaque est situé à 30 à 60 % à partir d'une portion d'extrémité proximale du ballonnet.
